# EUROPEAN PATENT APPLICATION

(11) **EP 1 956 007 A1**
(43) Date of publication of application: **13.08.2008**
(21) Application number: 06833883.9
(22) Date of filing: 01.12.2006
(51) Int. Cl.: C07D 235/08, C09K 11/06, H01L 51/50

(54) **NITROGENOUS HETEROCYCLIC DERIVATIVE AND ORGANIC ELECTROLUMINESCENCE DEVICE MAKING USE OF THE SAME**

(30) Priority: 02.12.2005 JP 2005349170
(71) Applicant: Idemitsu Kosan Co., Ltd., Chiyoda-ku Tokyo 100-8321 (JP)
(72) Inventor: YAMAMOTO, Hiroshi, Chiba, 299-0293 (JP); MATSUURA, Masahide, Chiba, 299-0293 (JP); KUBOTA, Mineyuki, Chiba, 299-0293 (JP); KAWAMURA, Masahiro, Chiba, 299-0293 (JP)
(74) Representative: Gille Hrabal Struck Neidlein Prop Roos
(86) International application number: PCT/JP2006/324113
(87) International publication number: WO 2007/063993

(57) **Abstract**

A novel heterocyclic derivative containing nitrogen having specific structure, and an organic EL device comprising at least one organic thin film layer comprising a light emitting layer interposed between a pair of electrodes consisting of an anode and a cathode in which at least one layer of the organic thin film layers comprises the novel heterocyclic derivative containing nitrogen singly or as its mixture component, are provided. By applying the heterocyclic derivative containing nitrogen to at least one organic layer, an organic EL device exhibits excellent emission luminance and current efficiency even at low electric voltage.

## Description

### TECHNICAL FIELD

The present invention relates to a novel heterocyclic derivative containing nitrogen, a material for an organic electroluminescence device ("electroluminescence" will be occasionally referred to as "EL", hereinafter) using the same and an organic electroluminescence device, in particular, to an organic EL device exhibiting excellent current efficiency even at a low electric voltage by employing a heterocyclic derivative containing nitrogen, which is useful as a construction component of the organic EL device, and is employed as at least a layer of organic layers thereof.

### BACKGROUND ART

An organic electroluminescence (EL) device using an organic substance has been taken to be a hopeful view on an application for a device of a large-sized full color display which is a solid light-emitting type and at a reasonable price: therefore, a variety of research and development have been carried out. Generally, an EL device comprises a light-emitting layer and a pair of counter electrodes interposing the layer. Emission of light is the following phenomenon: when an electric field is applied to the both electrodes, electrons are injected form a cathode and holes are injected from an anode. In addition, the electrons are recombined with holes in a light-emitting layer to produce an excited state. The energy, which is generated when the excited state returns to a ground state, is released as light.
Conventional organic EL devices require a higher driving voltage, and exhibits lower emission luminance and current efficiency in comparison with inorganic light-emitting diodes. In addition, deterioration of characteristic features is remarkable so that their practical uses have not been realized. Although recent organic EL devices have been improved gradually, higher emission luminance and higher current efficiency at a lower electric voltage are required.
So as to solve the above, for example, a device using a compound having a benzimidazole structure as a light-emitting material is disclosed and it emits light having luminance of 200 cd/m² at an electric voltage of 9V in the patent literature 1. In addition, compounds having a benzimidazole ring and a anthracene framework are described in the patent literature 2. However, organic EL devices have been required to have further improved emission luminance and current efficiency than the devices using these compounds.

Patent literature 1: U.S.P 5,645,948
Patent literature 2: Japanese Patent Application Laid-open No. 2002-38141

### DISCLOSER OF INVENTION

### [Problems to be overcome by the Invention]

The present invention has been made to overcome the above problems and provides a novel heterocyclic derivative containing nitrogen useful as a construction component for an organic EL device and has an objective for realizing the organic EL device having excellent emission luminance and current efficiency even at a low electric voltage by employing a heterocyclic derivative containing nitrogen as at least a layer of organic layers.

### [Means for overcoming the Problems]

As a result of intensive researches and studies to achieve the above objective by the present inventors, it was found that employing a novel heterocyclic derivative containing nitrogen having a specific structure as at least a layer of organic layers enables to provide the organic EL device having a excellent current efficiency at a low electric voltage.

Namely, the present invention provides a heterocyclic derivative containing nitrogen represented by the following general formula (1).

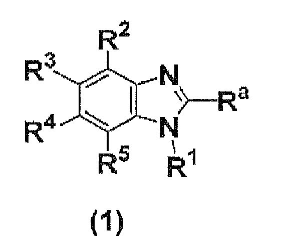

wherein, R¹ to R⁵ represents a hydrogen atom, a substituted or unsubstituted aryl group having 5 to 60 ring atoms, a substituted or unsubstituted heteroaryl group having 5 to 60 ring atoms, a substituted or unsubstituted pyridyl group, a substituted or substituted quinolyl group, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group,
R^{a} represents a substituted or unsubstituted alkyl group having 1 to 50 carbon atoms, adjacent groups within R² to R⁵ may bond each other to form an aromatic ring and at least one of R¹ to R⁵ represents a substituent represented by the following general formula (2).

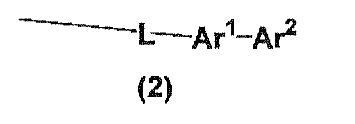

wherein, L represents a substituted or unsubstituted arylene group having 6 to 60 carbon atoms, a substituted or unsubstituted pyridinylene group, a substituted or unsubstituted quinolinylene group, or a substituted or unsubstituted fluorenylene group,
Ar¹ represents a substituted or unsubstituted arylene group having 6 to 60 carbon atoms, a substituted or unsubstituted pyridinylene group, or a substituted or unsubstituted quinolinylene group,
Ar² represents a hydrogen atom, a substituted or unsubstituted aryl group having 5 to 60 ring atoms, a substituted or unsubstituted pyridyl group, a substituted or unsubstituted quinolyl group, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group.

In addition, the present invention provides an organic EL device comprising at least a layer, which contains an aforementioned heterocyclic derivative containing nitrogen singly or as a component of a mixture thereof, of the organic thin film layers in an organic EL device comprising at least one of organic thin film layers including a light emitting layer interposed between a pair of electrodes consisting of a cathode and an anode.

### [The Effect of the Invention]

A heterocyclic derivative containing nitrogen of the present invention and an organic EL device using the same exhibit excellent current efficiency even at a low electric voltage and distinguished electron transportability.

### THE PREFERRED EMBODIMENT TO CARRY OUT THE INVENTION

Namely, the present invention provides a heterocyclic derivative containing nitrogen represented by the general formula (1):

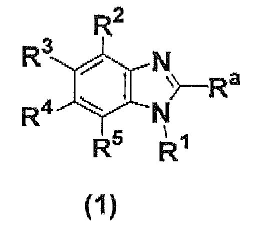

wherein, R¹ to R⁵ represents a hydrogen atom, a substituted or unsubstituted aryl group having 5 to 60 ring atoms, a substituted or unsubstituted heteroaryl group having 5 to 60 ring atoms, a substituted or unsubstituted pyridyl group, a substituted or substituted quinolyl group, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group,
R^{a} represents a substituted or unsubstituted alkyl group having 1 to 50 carbon atoms, and adjacent groups within R² to R⁵ may bond each other to form a aromatic ring and at least one of R¹ to R⁵ represents a substituent represented by the following general formula (2).

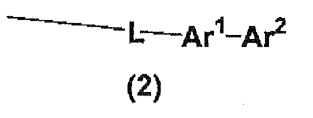

wherein, L represents a substituted or unsubstituted arylene group having 6 to 60 carbon atoms, a substituted or unsubstituted pyridinylene group, a substituted or unsubstituted quinolinylene group, or a substituted or unsubstituted fluorenylene group,
Ar¹ represents a substituted or unsubstituted arylene group having 6 to 60 carbon atoms, a substituted or unsubstituted pyridinylene group, or a substituted or unsubstituted quinolinylene group,
Ar² represents a hydrogen atom, a substituted or unsubstituted aryl group having 5 to 60 ring atoms, a substituted or unsubstituted pyridyl group, a substituted or unsubstituted quinolyl group, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group.

The present invention provides a heterocyclic derivative containing nitrogen which is a compound represented by the general formula (1) which is a compound represents by the following general formula (1-a) or (1-b).

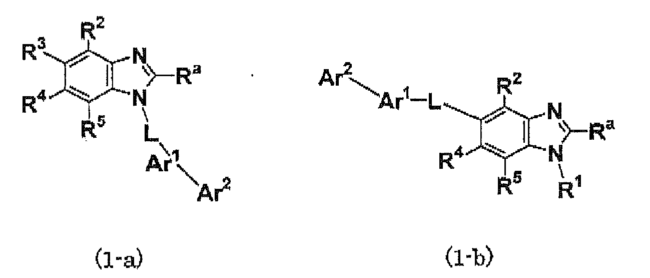

In the above general formulae (1-a) and (1-b), R¹ to R⁵, R^{a}, L, Ar¹ and Ar² each represents the same with all of the aforementioned general formula (1) respectively. In the above general formulae (1-a) and (1-b), a pair of adjacent groups within R² to R⁵ may bond each other to form an aromatic ring.

An aryl group and a heteroaryl group of the above R¹ to R⁵ and Ar² include ,for example, phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl) phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, fluoranthenyl group, fluorenyl group, 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acriclinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrole-5-yl group, 3-methylpyrrole-1-yl group, 3-methylpyrrole-2-yl group, 3-methylpyrrole-4-yl group, 3-methylpyrrole-5-yl group, 2-t-butylpyrrole-4-yl group, 3-(2-phenylpropyl)pyrrole-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group and the like.
Among those, phenyl group, naphthyl group, biphenyl group, anthracenyl group, phenathryl group, pyrenyl group, chrysenyl group, fluoranthenyl group and fluorenyl group are preferable.

A substituted or unsubstituted alkyl group having 1 to 50 carbon atoms of the aforementioned R¹ to R⁵, Ar² and R^{a} includes methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamanthyl group, 2-adamanthyl group, 1-norbornyl group, 2-norbornyl group and the like.

Example of a substituted or unsubstituted cycloalkyl group having 3 to 50 carbon atoms of the aforementioned R¹ to R⁵ and Ar² includes cyclopropyl group, cyclobutyl group, cyclopentyl group, cyclohexyl group, 4-methylcyclohexyl group, 1-adamantyl group, 2-adamantyl group, 1-norbornyl group, 2-norbornyl group and the like.
Example of a substituted or unsubstituted aralkyl group having 6 to 50 ring atoms of the aforementioned R¹ to R⁵ and Ar² includes benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

A substituted or unsubstituted alkoxy group having 1 to 50 carbon atoms of the aforementioned R¹ to R⁵ and Ar² means a group represented by -OY, and example for Y includes the similar groups to the aforementioned alkyl groups.
A substituted or unsubstituted aryloxy group having 5 to 50 ring atoms of the aforementioned R¹ to R⁵ and Ar² means a group represented by -OY', and example for Y' includes the similar groups to the aforementioned aryl groups.
A substituted or unsubstituted arylthio group having 5 to 50 ring atoms of the aforementioned R¹ to R⁵ and Ar² means a group represented by -SY', and example for Y' includes the similar groups to the aforementioned aryl groups.
A substituted or unsubstituted alkoxycarbonyl group having 1 to 50 carbon atoms of the aforementioned R¹ to R⁵ and Ar² means a group represented by -COOY, and example for Y includes the similar groups to the aforementioned alkyl groups.
Example of an aryl group of an amino group substituted by a substituted or unsubstituted aryl group having 5 to 50 ring atoms of the aforementioned R¹ to R⁵ and Ar² includes the similar groups to the aforementioned aryl groups.
Example of a halogen atom of the aforementioned R¹ to R⁵ and Ar² includes fluorine atom, chlorine atom, bromine atom, iodine atom and the like.

In the general formulae of (1), (1-a) and (1-b), a pair of adjacent groups within R² to R⁵ may bond each other to form an aromatic ring. 5 member ring and 6 member ring thereof are preferable, and 6 member ring is particularly preferable.

An arylene group having 6 to 60 carbon atoms of L and Ar¹ means a bivalent group formed by removing a hydrogen atom from a substituent which is explained in the aryl groups of R¹ to R⁵ and Ar². Phenylene group, naphthylene group, biphenylene group, anthranylene group, phenanthrylene group, pyrenylene group, chrysenylene group, fluoranthenylene group, fluorenylene group are preferable.
A pyridinylene group and a quinolinylene group of L and Ar¹ mean a bivalent substituent formed by removing a hydrogen atom from the aforementioned pyridinyl group or quinolynyl group.
A fluorenylene group of L means a bivalent group formed by removing a hydrogen atom from the aforementioned fluorene group.

It is preferable when a heterocyclic derivative of the present invention is a material for an organic EL device, and it is more preferable when it is a light emitting material for an organic EL device, an electron injecting material for an organic El device or an electron transporting material for an organic EL device.
The heterocyclic derivatives represented by the general formula (1) of the present invention will be described more specifically with reference to specific examples as follows: however, the derivatives are not limited by the examples.

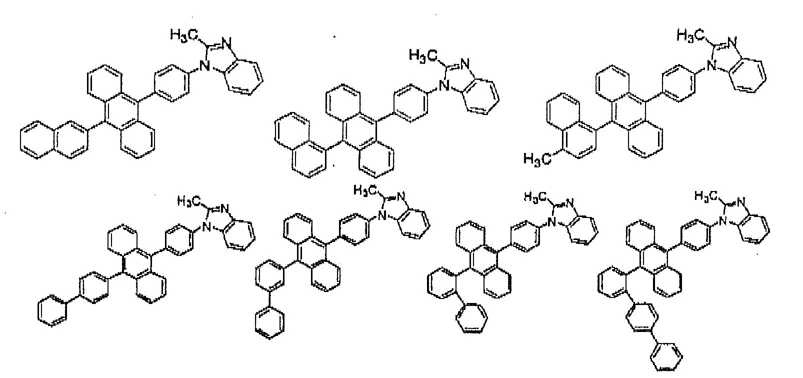

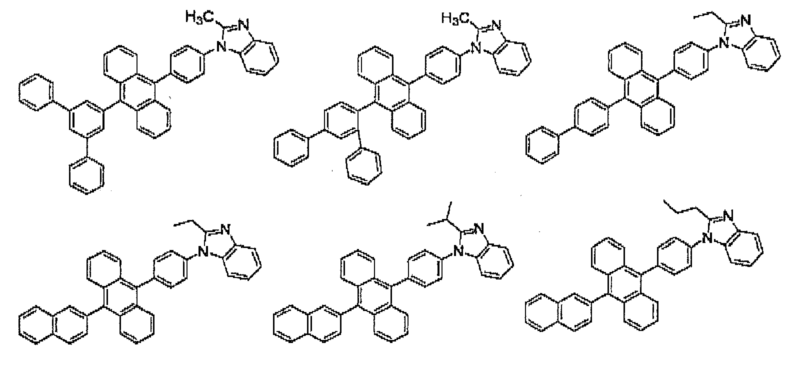

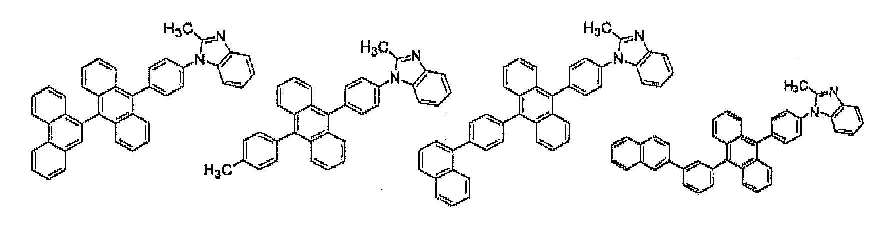

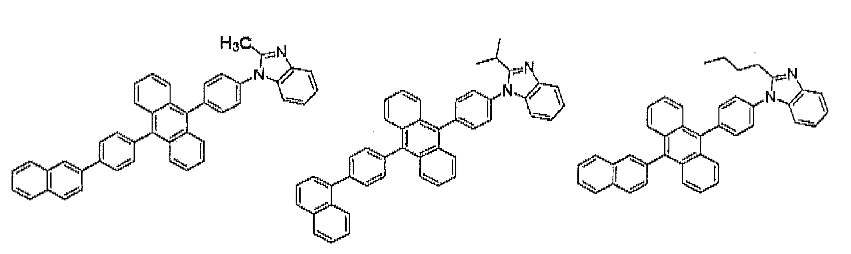

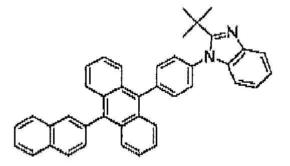

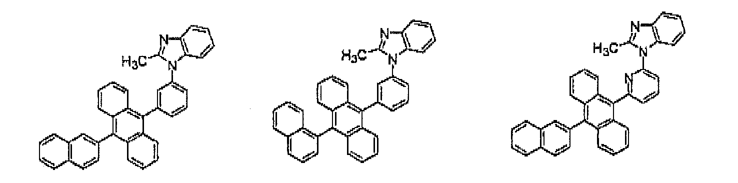

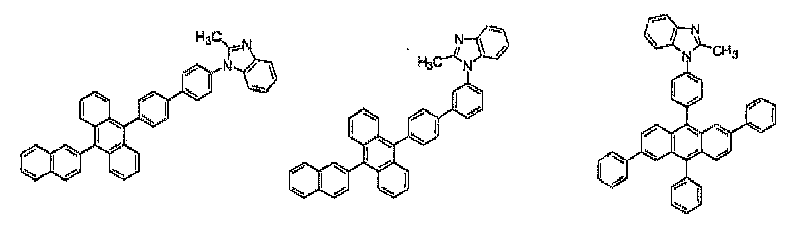

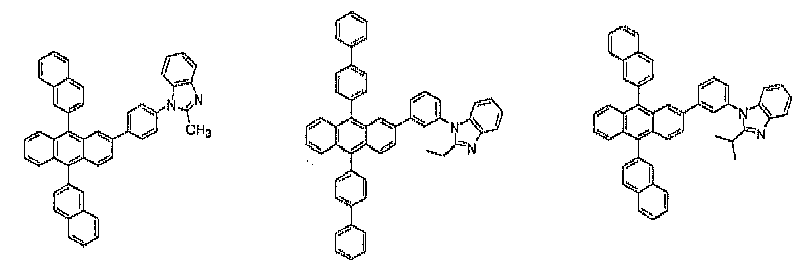

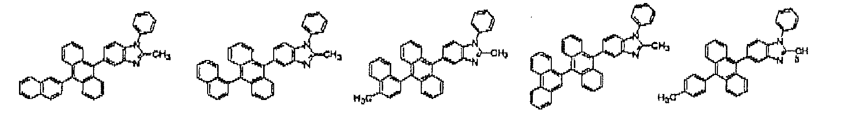

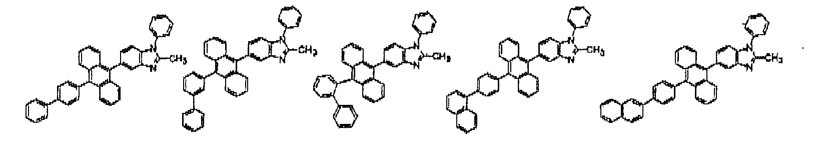

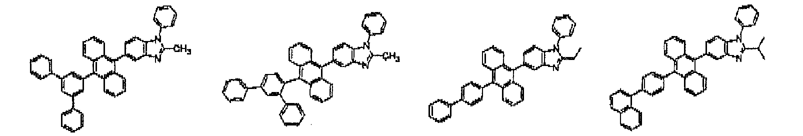

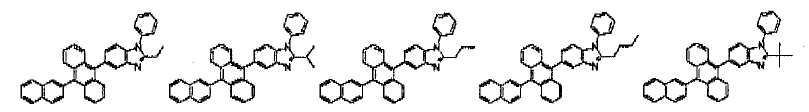

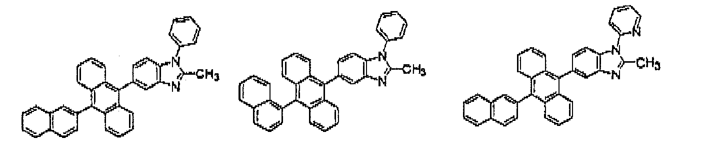

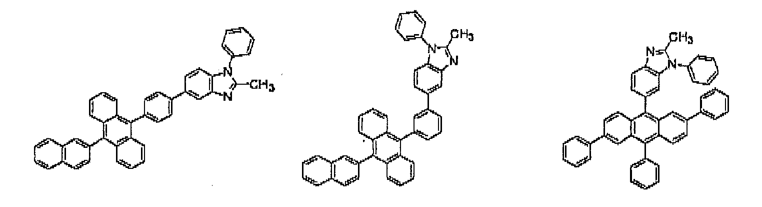

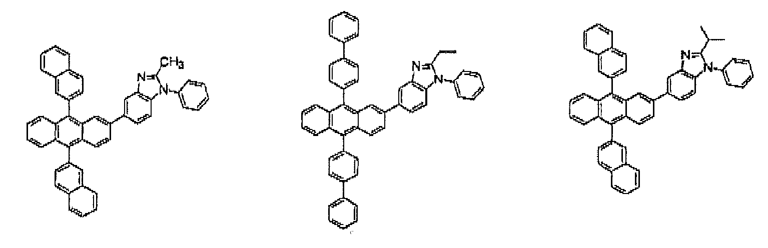

The following is an explanation of an organic EL device of the present invention.
An organic EL device of the present invention comprises at least a layer, which contains an aforementioned heterocyclic derivative containing nitrogen singly or as a component of a mixture thereof, of the organic thin film layers in an organic EL device comprising at least one of organic thin film layers including a light emitting layer interposed between a pair of electrodes consisting of a cathode and an anode.
It is preferable that an organic EL device of the present invention comprises the aforementioned organic thin film layer having an electron transporting layer and the electron transporting containing a heterocyclic derivative containing nitrogen of the present invention singly or as a component of mixture thereof. In addition, it is preferable that the aforementioned electron transporting layer contains a heterocyclic derivative containing nitrogen of the present invention as a main component thereof.
Further, it is preferable that a light emitting layer of an organic EL device of the present invention contains a heterocyclic derivative containing nitrogen, an arylamine compound and/or a styrylamine compound.
An arylamine compound includes a compound represented by the following general formula (A), and an styrylamine compound includes a compound represented by the following general formula (B)

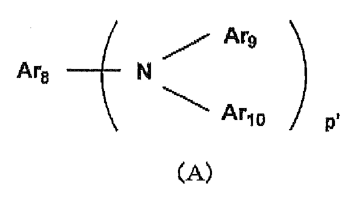

In the general formula (A), Arg represents a group selected from the group consisting of phenyl, biphenyl, terphenyl, stilbene and distyrylaryl, Ar₉ and Ar₁₀ each represents a hydrogen atom or an aromatic group having 6 to 20 carbon atoms, and Ar₉ and Ar₁₀ may be substituted. p' represents an integer of from 1 to 4. Further, it is preferable that Ar₉ and/or Ar₁₀ is substituted by a styryl group.
Here, a phenyl group, a naphtyl group, an anthracenyl group, a phenanthryl group, a terphenyl group, and the like are preferable for an aromatic group having 6 to 20 carbon atoms.

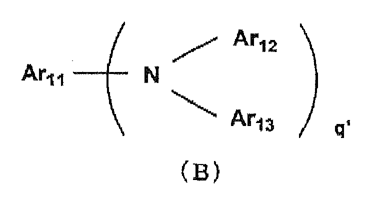

In the general formula (B), Ar₁₁ to Ar₁₃ is a substituted or unsubstituted aryl group having 5 to 40 ring carbon atoms. q' represents an integer of from 1 to 4.
Here, an aryl group having 5 to 40 ring atoms includes preferably phenyl, naphthyl, anthracenyl, phenanthryl, pyrenyl, coronyl, biphenyl, terphenyl, pyrrolyl, furanyl, thiophenyl, benzothiophenyl, oxadiazolyl, diphenylanthracenyl, indoryl, carbazolyl, pyridyl, benzoquinolyl, fluoranthenyl, acenaphthofluoranthenyl, stilbene, and the like. Further, an aryl group having 5 to 40 ring atoms may have a substituent which includes preferably an alkyl group having 1 to 6 carbon atoms such as ethyl group, methyl group, i-propyl group, n-propyl group, s-butyl group, t-butyl group, pentyl group, hexyl group, cyclopentyl group, cyclohexyl group and the like, an alkoxy group having 1 to 6 carbon atoms such as ethoxy group, methoxy group, i-propoxy group, n-propoxy group, s-butoxy group, t-butoxy group, pentoxy group, hexyloxy group, cyclopentoxy group, cyclohexyloxy group and the like, an aryl group having 5 to 40 ring atoms, an amino group substituted by an aryl group having 5 to 40 ring atoms, an ester group having an aryl group having 5 to 40 ring atoms, an ester group having an alkyl group having 1 to 6 carbon atoms, a cyano group, a nitro group and a halogen atom such as chlorine, bromine, iodine and the like.

The construction of the organic EL device of the present invention will be explained as follows:

### (1) The construction of the organic EL device

Typical examples of the construction of the organic EL device of the present invention include:
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) an anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode;
   and the like.
   Among those, the construction (8) is generally employed in particular; however, the construction of the organic EL device is not limited to those shown above as the examples.
   Although the heterocyclic derivatives containing nitrogen of the present invention may be employed for any of the above organic thin film layers, it is preferable that it is contained in a light emitting zone or a hole transporting zone among those construction elements, and it is particularly preferable that it is contained in an electron injecting layer, an electron transporting layer and a light emitting layer among those construction elements.

### (2) Substrate which transmits light

The organic EL device is produced on a substrate which transmits light. The substrate which transmits light, supports an organic EL device. It is preferable that it has a transmittance of light of 50% or greater in the visible region of 400 to 700 nm as well as flat and smooth thereof.
As the substrate which transmits light, for example, glass sheet and synthetic resin sheet are advantageously employed. Specific examples of the glass sheet include soda-lime glass, glass containing barium and strontium, lead glass, aluminosilicate glass, borosilicate glass, barium borosilicate glass and quartz. Specific examples of the synthetic resin sheet include sheet made of polycarbonate resins, acrylic resins, polyethylene terephthalate resins, polyether sulfide resins and polysulfone resins.

### (3) Anode

The anode in the organic EL device of the present invention covers a role of injecting holes into a hole transport layer or into a light emitting layer, and it is effective that the anode has a work function of 4.5 eV or greater. Specific examples of the material for the anode used in the present invention include indium tin oxide alloy (ITO), tin oxide (NESA), indium-zinc oxide (IZO), gold, silver, platinum, copper, etc.
The anode can be prepared by forming a thin film of the electrode material described above in accordance with a process such as a vapor deposition process or a sputtering process.
When the light emitted from the light emitting layer is observed through the anode, it is preferable that the anode has a transmittance of the emitted light greater than 10 %. It is also preferable that the sheet resistivity of the anode is several hundred Ω/□ or smaller. The thickness of the anode depends on a material, and is, in general, selected in the range of from 10 nm to 1µm and preferably in the range of from 10 to 200 nm.

### (4) Light emitting layer

The light emitting layer has the following (1) to (3) functions:
(1) The injecting function: the function of injecting holes from the anode or the hole injecting layer and injecting electrons from the cathode or the electron injecting layer when an electric field is applied;
(2) The transporting function: the function of transporting injected charges (electrons and holes) by the force of the electric field; and
(3) The light emitting function: the function of providing the field for recombination of electrons and holes and leading the recombination to the emission of light.
   Although there may be a difference between the capability of the holes being injected and the capability of the electrons being injected, and although there may be a grade about the transporting function expressed by mobility of the holes and the electrons, it is preferable to move charges of either ones.
   As the process for forming the light emitting layer, a well known process such as the vapor deposition process, the spin coating process and the LB process can be employed. It is preferable that a light emitting layer is a molecular deposit film particularly.
   Here, the molecular deposit film is defined as a thin film formed by deposit of a gas phase material compound or a thin film formed by solidification of a liquid or solution phase material compound. The molecular deposit film may be differentiated from a thin film (a molecular accumulation film) formed by the LB process, base on the differences between agglomeration structures and higher-order structures, and also the differences resulting from functionalities thereof.
   In addition, as shown in Japanese Patent Laid-open No. Shou 57(1982)-51781, to form a light emitting layer, a thin film may be formed in accordance with the spin coating and the like of the solution to be prepared by dissolving a binder such as resin and a material compound in solvent.
   In the present invention, any well known light emitting material other than a light emitting material consisting of a heterocyclic derivative containing nitrogen of the present invention may be optionally contained in the light emitting layer; or a light emitting layer containing other well known light emitting layer may be laminated with the light emitting layer containing the light emitting material comprising a heterocyclic derivative containing nitrogen of the present invention each in an extent of not obstructing to achieve the objective of the present invention respectively.

A light emitting material or a doping material to be used together with a heterocyclic derivative containing nitrogen includes, for example, anthracene, naphthalene, phenanthrene, pyrene, tetracene, coronene, chrysene, fluorescein, perylene, phthaloperylene, naphtaloperylene, perinone, phthaloperinone, naphthaperinone, diphenylbutadiene, tetraphenylbutadiene, coumarin, oxadiazole, aldazine, bisbenzooxazoline, bisstyryl, pyrazine, cyclopentadiene, quinoline metal complex, aminoquinoline metal complex, benzoquinoline metal comple, imine, diphenylethylene, vinylanthracene, diaminocarbazol, pyran, thiopyran, polymethine, merocyanine, imidazol chelated oxinoid compound, quinacridone, rubrene and fluorescent dye, but not limited thereto.

A preferable host material to be used together with a heterocyclic derivative containing nitrogen of the present invention includes compounds represented by the general formula (i) to (ix).

An asymmetric anthracene represented by the general formula (i):

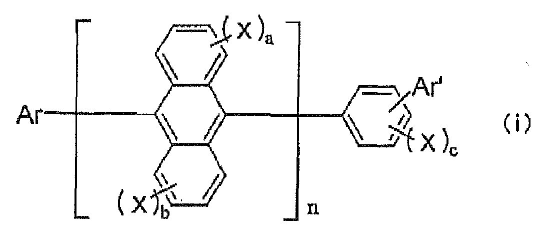

wherein, Ar represents a substituted or unsubstituted condensed aromatic group having 10 to 50 ring carbon atoms.
Ar' represents a substituted or unsubstituted aromatic group having 6 to 50 ring carbon atoms.
X represents a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a carboxyl group, a halogen atom, a cyano group, a nitro group and a hydroxyl group.
a, b and c each independently represents an integer of 0 to 4.
n represents an integer of 1 to 3. Further, in a case where n represents 2 or more, the plural group within "[ ]" may be the same with or different from each other.

An asymmetric mono-anthracene derivative represented by the general formula (ii):

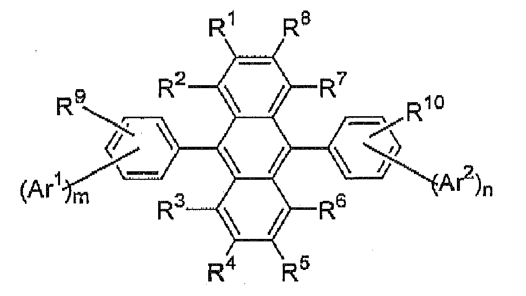

wherein, Ar¹ and Ar² each independently represents a substituted or unsubstituted aromatic ring group having 6 to 50 ring carbon atoms, and m and n each represents an integer of 1 to 4; however, in a case where m=n=1 and each bonding position of Ar¹ and Ar² to a benzene ring is mono-symmetrical each other, Ar¹ represents different from Ar², and when m or n represents an integer of 2 to 4, m is different from n.
R¹ to R¹⁰ each independently represents a hydrogen atom, a substituted or unsubstituted aromatic ring carbon group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 ring carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group and a hydroxyl group.

An asymmetric pyrene derivative represented the general formula (iii)

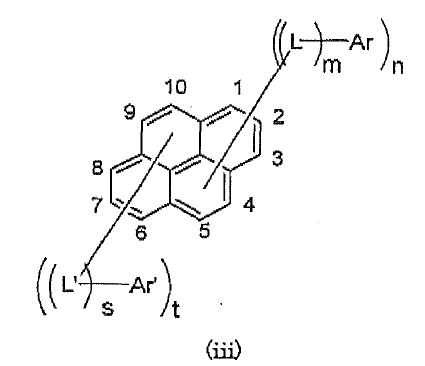

wherein, Ar and Ar' each independently a substituted or unsubstituted aromatic group having 6 to 50 ring carbon atoms.
L and L' each independently represents a substituted or unsubstituted phenylene group, a substituted or unsubstituted naphthalenylene group, a substituted or unsubstituted fluorenylene group or a substituted or unsubstituted dibenzosilolylene group.
m represents an integer of 0 to 2, n represents of an integer of 1 to 4, s represents an integer of 0 to 2 and t represents an integer of 0 to 4.
Further, L or Ar bonds to any one of 1 to 5 position of pyrene, and L' or Ar' bonds to any one of 6 to 10 position thereof.
However, when n + t is an even number, Ar, Ar', L and L' satisfy a following requirement (1) or a requirement (2):
(1) Ar≠Ar' and/or L≠L' (wherein ≠ means that each group has a different structure)
(2) when Ar = Ar' and L = L'
   (2-1) m≠s and/or n≠t, or
   (2-2) when m = s and n = t,
      (2-2-1) both L and L' or pyrene respectively bond to a different position of Ar and Ar', or
      (2-2-2) both L and L' or pyrene respectively bond to the same position of Ar and Ar',
      excluding a case where both L and L' or both Ar and Ar' bond respectively to 1 and 6, or 2 and 7 positions thereof.

An asymmetric anthracene derivative represented by the following general formula (iv):

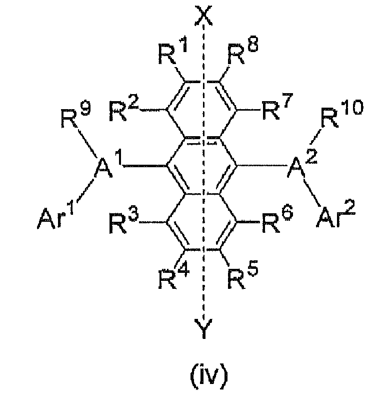

wherein, A¹ and A² each independently represents a substituted or unsubstituted condensed aromatic ring group having 10 to 20 ring carbon atoms.
Ar¹ and Ar² each independently represents a hydrogen atom, or a substituted or unsubstituted aromatic ring group having 6 to 50 ring carbon atoms.
R¹ to R¹⁰ each independently represents a hydrogen atom, a substituted or unsubstituted aromatic ring group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted cycloalkyl group, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted axylthio group having 5 to 50 ring atoms, a substituted or unsubstituted alkoxycarbonyl group having 1 to 50 carbon atoms, a substituted or unsubstituted silyl group, a carboxyl group, a halogen atom, a cyano group, a nitro group or a hydroxyl group.
Ar¹, Ar², R⁹ and R¹⁰ each may be more than one, and two adjacent groups thereof may form a saturated or unsaturated ring structure.
However, it is excluded a case where the groups at 9 and 10 positions of anthracene at the core are symmetrical at x-y axis of symmetry and bond each other.

An anthracene derivative represented by the general formula (v):

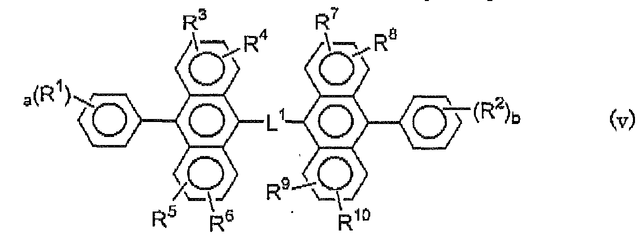

wherein, R¹ to R¹⁰ each independently represents a hydrogen atom, an alkyl group, a cycloalkyl group, an aryl group which may be substituted, an alkoxyl group, an aryloxy group, an alkylamino group, an alkenyl group, an arylamino group or a heterocyclic group which may be substituted. a and b each represents an integer of 1 to 5, and when a or b is 2 or more, R¹s or R²s may be the same with or different from each other, additionally R¹s or R²s may bond each other to form a ring; each pair of R³ and R⁴, R⁵ and R⁶, R⁷ and R⁸, and/or R⁹ and R¹⁰ may bond each other to form a ring. L¹ represents a single bond, -O-, -S-, -N(R)-, an alkylene group or an arylene group; wherein R represents an alkyl group, or an aryl group which may be substituted.

An anthracene derivative represented by the general formula (vi):

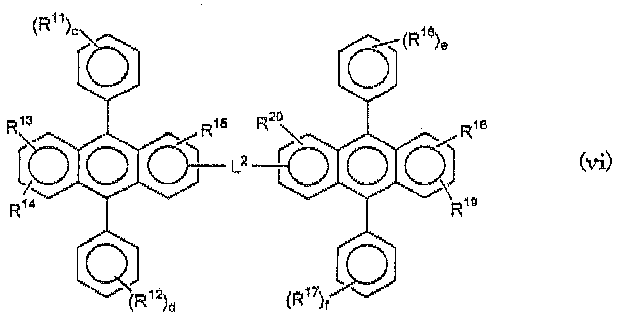

wherein, R¹¹ to R²⁰ each independently represents a hydrogen atom, an alkyl group, a cycloalkyl group, an aryl group, an alkoxyl group, an aryloxy group, an alkylamino group, an arylamino group or a heterocyclic group which may be substituted, c, d, e and f each represents an integer of 1 to 5, and when c, d, e and/or f are 2 or more, R¹¹s, R¹²s, R¹⁶s or R¹⁷s may be the same with or different from each other, additionally each pair of R¹¹s, R²s, R¹⁶s or R¹⁷s may bond each other to form a ring; and R¹³ and R¹⁴, and/or R¹⁸ and R¹⁹ may bond each other to form a ring. L² represents a single bond, -O-, -S-, -N-(R)-, an alkylene group or an arylene; wherein R represents an alkyl group, or an aryl group which may be substituted.

A spirofluorene derivative represented by the general formula (vii):

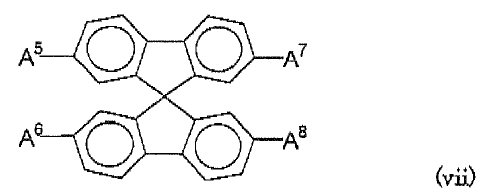

wherein, A⁵ to A⁸ each independently represented a substituted or unsubstituted biphenyl group or a substituted or unsubstituted naphthyl group.

A compound containing a condensed ring represented by the general formula (viii):

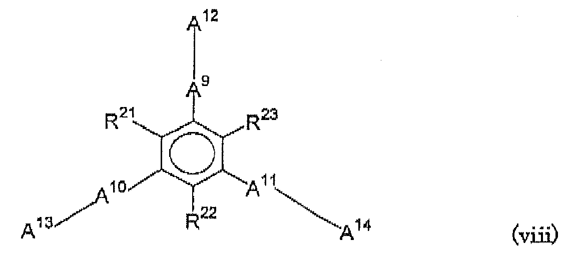

wherein, A⁹ to A¹⁴ represented the same with aforementioned, and R²¹ to R²³ each independently represents a hydrogen atom, an alkyl group having 1 to 6 carbon atoms, a cycloalkyl group having 3 to 6 carbon atoms, an alkoxyl group having 1 to 6 carbon atoms, an aryloxy group having 5 to 18 ring carbon atoms, an aralkyloxy group having 7 to 18 carbon atoms, an arylamino group having 5 to 16 carbon atoms, a nitro group, a cyano group, an ester group having 1 to 6 carbon atoms or a halogen atom, and at least one of A⁹ to A¹⁴ represents a condensed aromatic ring comprising 3 or more rings.

A fluorene compound represented by the general formula (ix):

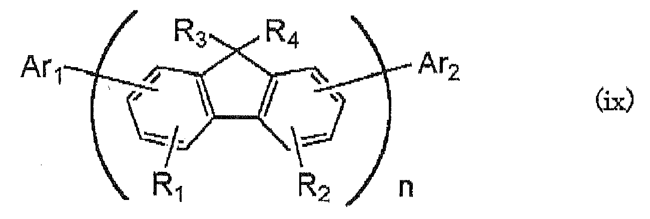

wherein, R₁ and R₂ each independently represents a hydrogen atom, a substituted or unsubstituted alkyl group, a substituted or unsubstituted aralkyl group, a substituted or unsubstituted aryl group, a substituted or unsubstituted heterocyclic group, a substituted amino group, a cyano group or a halogen atom. R₁s and R₂s bonding to a different fluorene group may be the same with or different from each other, and R₁ and R₂ bonding to the same fluorene group may be the same with or different from each other. R₃ and R₄ each independently represents a hydrogen atom, a substituted or unsubstituted alkyl group, a substituted or unsubstituted aralkyl group, a substituted or unsubstituted aryl group, or a substituted or unsubstituted heterocyclic group, and R₃s and R₄s bonding to a different fluorene group may be the same with or different from each other, and also both R₃ and R₄ bonding to the same fluorene group may be the same with or different from each other. Ar₁ and Ar₂ each independently represents a substituted or unsubstituted condensed polycyclic aromatic group consisting of benzene rings of 3 or more or a substituted or unsubstituted condensed polycyclic heterocyclic group comprising of benzene rings and heterocylces of 3 or more in total, and Ar₁ and Ar₂ may be the same with or different from each other. n represents an integer of 1 to 10.

Among the above host materials, an anthracene derivative is preferable and a mono-anthracene derivative is more preferable, further an asymmetric anthracene is particularly preferable.
In addition, a phosphorescent compound may be employed as a light emitting material for dopant. A compound containing a carbazole ring for a host material is preferable as a phosphorescent compound. Although a dopant is a compound which is able to emit light from triplet exciton and is not limited as long as emitting light from triplet exciton, it is preferable that a metal complex contains at least a metal selected from a group consisting of Ir, Ru, Pd, Pt, Os and Re.
A suitable host for phosphorescence comprising a compound containing a carbazole ring is a compound having a function of making a phosphorescent compound to emit light as a result of energy transfer from its excited state to the phosphorescent compound. With regard to the host compound, any compound being able to transfer exciton energy to the phosphorescent compound may be selected, without particularly restricted, for the purpose as appropriate. Any heterocylce excluding a carbazole ring may be contained.

Specific examples of the host compound include a carbazole derivative, a triazole derivative, an oxazole derivative, an oxadiazole derivative, an imidazole derivative, a polyarylalkane derivative pyrazoline derivative, a pyrazolone derivative, a phenylenediamine derivative, an arylamine derivative, a chalcone derivative substituted by amine, a styrylanthracene derivative, a fluorenone derivative, hydrazone derivative, a stilbene derivative, a silazane derivative, an aromatic tertiary amine compound, a styrylamine compound, an aromatic dimethylidene type compound, a porphyrin type compound, an anthtaquinodimethane derivative, an anthrone derivative, a diphenylquinone derivative, a thiopyran dioxide derivative, a carbodiimide derivative, a fluorenylidene methane derivative, a distyrylpyrazine derivative, heterocyclic tetracarboxylic anhydride such as a naphthalene perylene derivative, a phthalocyanine derivative, a metal complex of 8-quinolinol derivative, a metallo-phthalocyanine, various metal complexes represented by a metal complex having a ligand of benzoxazole or benzothiazole, a polysilane based compound, a conductive oligomer such as a poly(N-vinylcarbazole) derivative, an aniline based copolymer, a thiophene oligomer, a polythiophene derivative, a polyphenylene derivative, a polyphenylene vinylene derivative, a polyfluorene derivative and the like. The host compounds may be used singly or in combination of two or more.
More specific examples include the following;

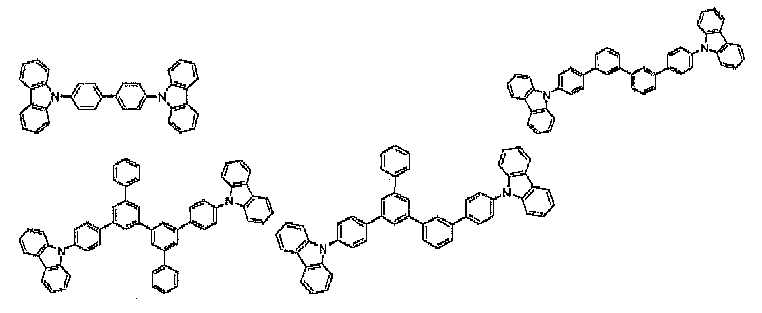

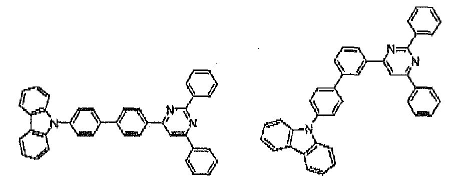

The phosphorescent dopant is a compound capable of emitting light from the triplet exciton. Although it is not restricted as long as it emits light from the triplet exciton, it is preferable that a metal complex comprises at least a metal selected from the group of Ir, Ru, Pd, Pt, Os and Re, and a porphyrin metal complex or an orthometalized metal complex is particularly preferable. As the porphyrin metal complex, a porphyrin platinum complex is preferable. The phosphorescent compound may be employed singly or in combination of two or more.
Although there are various ligands to form the orthometalized metal complex, preferred includes 2-phenylpyridine derivatives, 7,8-benzoquinoline derivatives, 2-(2-thienyl)pyridine derivatives, 2-(1-naphthylpyridine derivatives, 2-phenylquinoline derivatives and the like. The derivatives may have substituent as occasion demands. In particular, the derivatives having a fluorinated compound or a trifluoromethyl group are preferable for a dopant emitting blue hue color. In addition, a ligand other than the above ligand such as acetylacetonate and picnic acid may be contained as an auxiliary ligand.
The amount of the phosphorescent dopant in the light emitting layer may be selected for the objective as appropriate without particularly restricted, and for example, it may be selected in the range of from 0.1 to 70 % by mass, preferably in the range of from 1 to 30 % by mass. The emission is faint and the advantage is not demonstrated when the amount is less than 0.1 % by mass. The concentration quenching becomes noticeable so that the device performance is deteriorated when the amount is more than 70 % by mass.
Further, the light emitting layer may contain a hole transporting material, a electron transporting material or a polymer binder as appropriate.
In addition, the thickness of the light emitting layer is, in general, selected in the range of from 5 to 50 nm, preferably in the range of from 7 to 50 nm and more preferably in the range of from 10 to 50 nm. It is resulted in difficult to form the light emitting layer and to control chromaticity thereof when the thickness is less than 5 nm, and it may be resulted in danger of increasing driving voltage when it is more than 50 nm.

### (5) Hole injecting / transporting layer (Hole transporting zone)

The hole injecting / the hole transporting layer is a layer which assists injection of holes into the light emitting layer and transport the holes to the light emitting zone. The layer exhibits a great mobility of holes and, in general, has an ionization energy as small as 5.5 eV or smaller. For the hole injecting / the hole transporting layer, a material which transports holes to the light emitting layer at a small strength of the electric field is preferable. A material which exhibits, for example, a mobility of holes of at least 10⁻⁴ cm²/V· sec under application of an electric field of from 10⁴ to 10⁶ V/cm is preferable.
When a heterocyclic derivative containing nitrogen of the present invention is employed for hole transporting zone, it may be used singly or in combination with other material to form a hole injecting / transporting layer.
With regard to the material which may be employed for forming the hole injecting / transporting layer in combination with the heterocyclic derivatives, any material having the foregoing preferable properties is employed without particularly restricted, and any arbitrary material selected from conventional material commonly used as a charge transporting material for the holes in photoconducting materials and well known material employed for the hole injecting / transporting layer in the EL device may be employed.

Further examples include triazole derivatives (refer to United States Patent No. 3,112,197, etc.), oxadiazole derivatives (refer to United States Patent No. 3,189,447, etc.), imidazole derivatives (refer to Japanese Examined Patent KOKOKU No. Shou 37-16096, etc.), poly arylalkane derivatives (refer to United States Patent Nos. 3,615,402, 3,820,989 and 3,542,544, Japanese Examined Patent KOKOKU Nos. Shou 45-555 and Shou 51-10983, Japanese Unexamined Patent Application Laid-Open Nos. Shou 51-93224, Shou 55-17105, Shou 56-4148, Shou 55-108667, Shou 55-156953, Shou 56-36656, etc.), pyrazoline derivatives and pyrazolone derivatives (refer to U.S Patent Nos. 3,180,729 and 4,278,746, Japanese Unexamined Application Patent Laid-Open Nos. Shou 55-88064, Shou 55-88065, Shou 49-105537, Shou 55-51086, Shou 56-80051, Shou 56-88141, Shou 57-45545, Shou 54-112637, Shou 55-74546, etc.), phenylenediamine derivatives (refer to U.S Patent No. 3,615,404, Japanese Examined Patent KOKOKU Nos. Shou 51-10105, Shou 46-3712 and Shou 47-25336, Japanese Unexamined Patent Application Laid-Open Nos. Shou 54-53435, Shou 54-110536, Shou 54-119925, etc.), arylamine derivatives (refer to U.S Patent Nos. 3,567,450, 3,180,703, 3,240,597, 3,658,520, 4,232,103, 4,175,961 and 4,012,376, Japanese Examined Patent KOKOKU Nos. Shou 49-35702 and Shou 39-27577, Japanese Unexamined Patent Application Laid-Open Nos. Shou 55-144250, Shou 56-119132 and Shou 56-22437, West German Patent No. 1,110,518, etc.), chalcone derivatives which is substituted with amino group (refer to U.S Patent No. 3,526,501, etc.), oxazole derivatives (disclosed in U.S Patent No. 3,257,203, etc.), styryl anthracene derivatives (refer to Japanese Unexamine Patent Application laid-Open No. Shou 56-46234, etc.), fluorenone derivatives (refer to Japanese Unexamined Patent Application Laid-Open No. Shou 54-110837, etc.), hydrazone derivatives (refer to U.S Patent Nos. 3,717,462, Japanese Unexamined Patent Application Laid-Open Nos. Shou 54-59143, Shou 55-52063, Shou 55-52064, Shou 55-46760, Shou 55-85495, Shou 57-11350, Shou 57-148749, Hei 2-311591, etc.), stilbene derivatives (refer to Japanese Unexamined Patent Application Laid-Open Nos.Shou 61-210363, Shou 61-228451, Shou 61-14642, Shou 61-72255, Shou 62-47646, Shou 62-36674 , Shou 62-10652, Shou 62-30255, Shou 60-93455, Shou 60-94462, Shou 60-174749, Shou 60-175052, etc.), silazane derivatives (U.S Patent No. 4,950,950), polysilane-based copolymers (Japanese Unexamined Patent Application Laid-Open No. Hei 2-204996), aniline-based copolymers (Japanese Unexamined Patent Application Laid-Open No. Hei 2-282263), an electroconductive polymer oligomer which is disclosed in Japanese Unexamined Patent Application Laid-Open No Hei 1-211399 (particularly, thiophene oligomer), etc.

With regard to the material of the hole injecting / transporting layer, the above materials are also employable, however, porphyrin compounds (published in Japanese Unexamined Patent Application Laid-Open Nos. Shou 63-2956965, etc.), aromatic tertiary amine compounds and styryl amine compounds (refer to U.S Patent No. 4,127,412, Japanese Unexamined Patent Application Laid-Open Nos. Shou 53-27033, Shou 54-58445, Shou 54-149634, Shou 54-64299, Shou 55-79450, Shou 55-144250, Shou 56-119132, Shod 61-295558, Shou 61-98353, Shou 63-295695, etc.) are preferable and the aromatic tertiary amine compounds are particularly preferable.
Further examples include, for example, 4,4'-bis(N-(1-naphthyl)-N-phenylamino)biphenyl (abbreviated as NPD hereinafter) having 2 condensed aromatic rings in its molecule described in U.S Patent No. 5,061,569, 4,4',4"-tris(N-(3-methylphenyl)-N-phenylamino)triphenylamine (abbreviated as MTDATA hereinafter) made by connecting three triphenyl amine units to form a star burst type and the like.
Further, in addition to the aforementioned aromatic dimethylidyne based compounds described as a material for the light emitting layer, inorganic compound such as p-type Si, p-type SiC or the like is employable as the material for the hole injecting / transporting layer.

To form the hole injecting / the hole transporting layer, a thin film may be formed from a heterocyclic derivative containing nitrogen of the present invention in accordance with a well known process such as the vacuum vapor deposition process, the spin coating process, the casting process and the LB process. Although the thickness of the hole injecting / the hole transporting layer is not particularly limited, the thickness is usually from 5 nm to 5 µm. When the hole injecting / transporting layer comprises a heterocyclic derivative containing nitrogen of the present invention in the hole transporting zone thereof, the hole injecting / transporting layer may be constructed by a layer comprising at least one of the aforementioned materials or by laminating a hole injecting / transporting layer comprising a different compound other than the aforementioned hole injecting / transporting layer.
An organic semiconductor layer may be provided to assist to inject the holes or to inject the electrons into the light emitting layer, and it is preferable for the organic semiconductor layer to have a electric conductivity of 10⁻¹⁰ S/cm or greater. With regard to a material for the organic semiconductor layer, electroconductive oligomers such as an oligomer having thiophene, an oligomer having arylamine disclosed in Japanese Unexamined Patent Application Laid-Open No. 8-193191 and the like, electroconductive dendrimers such as a dendrimer having an arylamino dendrimer are employable.

### (6) Electron injecting / transporting layer

Next, the electron injection / transporting layer in the organic EL device of the present invention is a layer which assists injection of electrons into the light emitting layer and transportation thereof to the light emitting zone, and exhibits a great mobility of electrons and electron affinity of 2.5 eV or larger generally. For such electron injection / transporting layer, it is preferable that a material transports electrons to a light emitting material at lower electric field strength. In addition, a mobility of electrons of at least 10⁻⁶ cm²/V.sec under application of an electric field of from 10⁴ to 10^{G} V/cm is preferable.
When a heterocyclic derivative containing nitrogen of the present invention is employed for electron transporting zone, it may be used singly or in combination with other material to form an electron injecting / transporting layer.
With regard to the material which may be employed for forming the electron injecting / transporting layer in combination with a heterocyclic derivative containing nitrogen of the present invention, any material having the foregoing preferable properties is employed without particularly restricted, and any arbitrary material selected from conventional material commonly used as a charge transporting material for the electrons in photoconducting materials and well known material employed for the electron injecting / transporting layer in the EL device may be employed.
Among the electron injecting layers, an adhesion improving layer is a layer made of a material exhibiting excellent adhesion with the cathode. In the organic EL device of the present invention, it is preferable that a compound of the present invention is employ as an electron injecting / transporting layer, an adhesion improving layer.

As a preferable organic EL device of the present invention, it has a reductive dopant added in either the electron transporting zone or an interfacial zone between the cathode and the organic layer. In the present invention, it is preferable that the organic EL device employs the compound of present invention containing a reductive dopant. The reductive dopant used in the present invention is defined as a substance which reduces the electron transporting compound. Therefore, various compounds may be employed if they have a certain level of reduction capability. Examples of the preferable reductive dopant include at least one compound selected from the group comprising alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals and organic complexes of rare earth metals.
Examples of the more preferable reductive dopant include at least one alkali metal selected from a group consisting of Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9eV), Sr (the work function: 2.0 to 2.5 eV) and Ba (the work function: 2.52eV); whose work function of 2.9 eV or smaller is particularly preferable. Among those, more preferable reductive dopants include at least one kind or more alkali metal selected from the group consisting of K, Rb and Cs, the latter Rb or Cs being farther more preferable and the last Cs being the most preferable. Those alkali metals have particularly high reducing capability, and only an addition of relatively small amount of them into an electron injection zone enables to achieve both improvement of emission luminance and lifetime extension of the organic EL device. Further, with regard to the reductive dopant with work function of 2.9 eV or smaller, a combination of two or more kinds of the alkali metal is also preferable, and particularly, combinations containing Cs, for example, combinations of Cs and Na, Cs and K, Cs and Rb, or Cs and Na and K are preferable. Containing Cs in combination enables to reveal reducing capability effectively, and the addition into the electron injection zone expects both improvement of emission luminance and lifetime extension of the organic EL device.

In the organic EL device of the present invention, an electron injecting layer formed with an insulating material or a semiconductor may be further sandwiched between the cathode and the organic thin film layer. The electron injecting layer effectively prevents leak in the electric current and improves the electron injecting capability. It is preferable that at least one metal compound selected from the group consisting of alkali metal chalcogenides, alkaline earth metal chalcogenide, alkali metal halides and alkaline earth metal halides is used as the insulating material. It is preferable that the electron injecting layer is constituted with the above alkali metal chalcogenide since the electron injecting capability can be improved. Preferable examples of the alkali metal chalcogenide include Li₂O, K₂O, Na₂S, Na₂Se and Na₂O. Preferable examples of the alkaline earth metal chalcogenide include CaO, BaO, SrO, BeO, BaS and CaSe. Preferable examples of the alkali metal halide include LiF, NaF, KF, LiCl, KCl, NaCl and the like. Preferable examples of the alkaline earth metal halide include fluorides such as CaF₂, BaF₂, SrF₂, MgF₂ and BeF₂ and halides other than the fluorides.
Examples of the semiconductor constituting the electron transporting layer include oxides, nitrides and oxide nitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron transporting layer is in the form of crystallite or amorphous insulating thin film. When the electron transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

### (7) Cathode

As the cathode for the organic EL device of the present invention, an electrode substance such as metal, alloy, electroconductive compound and those mixture having a small work function (4 eV or smaller) is employed. Examples of the electrode substance include sodium, sodium- potassium alloy, magnesium, lithium, magnesium-silver alloy, aluminum / aluminum oxide, aluminum-lithium alloy, indium, rare earth metal, and the like.
The cathode can be prepared by forming a thin film of the electrode material described above in accordance with a process such as the vapor deposition process and the sputtering process.
When the light emitted from the light emitting layer is observed through the anode, it is preferable that the anode has a transmittance of the emitted light greater than 10 %.
It is also preferable that the sheet resistivity of the anode is several hundred Ω / □ or smaller. The thickness of the anode is, in general, selected in the range of from 10 nm to 1 µm and preferably in the range of from 50 to 200 nm.

### (8) Insulating layer

An organic EL device tends to form defects in pixels due to leak and short circuit since an electric field is applied to ultra-thin films. To prevent the formation of the defects, a layer of an insulating thin film may be inserted between the pair of electrodes.
Examples of the material employed for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds may also be employed.

### (9) Process of producing an organic EL device

To produce an organic EL device of the present invention, for example, an anode, a light emitting layer and, where necessary, a hole injecting / transporting layer, and where necessary, an electron injecting / transporting layer may be formed in accordance with the aforementioned process using the aforementioned materials, and a cathode is formed in the last step. An organic EL device may be produced by forming the aforementioned layers in the order reverse to that described above, i,e., a cathode being formed in the first step and an anode in the last step.
An embodiment of the process for producing an organic EL device having a construction in which an anode, a hole injecting layer, a light emitting layer, an electron injecting layer and a cathode are disposed sequentially on a light-transmitting substrate will be described in the following.
On a suitable light-transmitting substrate, a thin film made of a material for the anode is formed in accordance with the vapor deposition process or the sputtering process so that the thickness of the formed thin film is 1 µm or smaller and preferably in the range of 10 to 200 nm. Then, a hole injecting layer is formed on the anode. The hole injecting layer can be formed in accordance with the vacuum vapor deposition process, the spin coating process, the casting process or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions in general are suitably selected in the following ranges: temperature of the deposition source: 50 to 450 degC; vacuum level: 10⁻⁷ to 10⁻³ Torr; deposition rate: 0.01 to 50 nm/second; temperature of the substrate: -50 to 300 degC, and film thickness: 5 nm to 5 µm; although the conditions of the vacuum vapor deposition are different depending on the employed compound (the material for the hole injecting layer) and the crystal structure and the recombination structure of the hole injecting layer to be formed.

Subsequently, the light-emitting layer is formed on the hole-injecting layer formed above. Also the formation of the light emitting layer can be made by forming the desired light emitting material into a thin film in accordance with the vacuum vapor deposition process, the sputtering process, the spin coating process or the casting process. The vacuum vapor deposition process is preferable because a uniform film can be easily obtained and the possibility of formation of pinholes is small. When the light emitting layer is formed in accordance with the vacuum vapor deposition process, in general, the conditions of the vacuum vapor deposition process can be selected in the same ranges as those described for the vacuum vapor deposition of the hole injecting layer although the conditions are different depending on the used compound.
Next, the electron injecting layer is formed on the light emitting layer formed above. Similarly to the hole injecting layer and the light emitting layer, it is preferable that the electron injecting layer is formed in accordance with the vacuum vapor deposition process since a uniform film should be obtained. The conditions of the vacuum vapor deposition can be selected in the same ranges as those for the hole injecting layer and the light emitting layer.
Although a heterocyclic derivative containing nitrogen of the present invention depends on that it is contained in a light emitting layer or a hole transporting layer, it may be vapor deposited together with other materials. In addition, when the spin coating process is employed, it may be contained therein by blending it with other materials.
An organic EL device is produced by laminating a cathode as the last step.
The anode is made of a metal and can be formed in accordance with the vacuum vapor deposition process or the sputtering process. It is preferable that the vacuum vapor deposition process is employed in order to prevent the lower organic layers from damages during the formation of the film.
In the above production of the organic EL device, it is preferable that the above layers from the anode to the cathode are formed successively while the production system is kept in a vacuum after being evacuated once.

The process for forming the layers in the organic EL device of the present invention is not particularly limited. A conventional process such as the vacuum vapor deposition process and the spin coating process can be used. The organic thin film layer comprising the compound represented by the foregoing general formula (1) used in the organic EL device of the present invention can be formed in accordance with the vacuum vapor deposition process, the molecular beam epitaxy process (the MBE process) or, using a solution prepared by dissolving the compound into a solvent, in accordance with a conventional coating process such as the dipping process, the spin coating process, the casting process, the bar coating process and the roller coating process.
The thickness of each layer in the organic thin film layer in the organic EL device of the present invention is not particularly limited. In general, an excessively thin layer tends to have defects such as pin holes, and an excessively thick layer requires a high applied voltage and results in decreasing the efficiency. Therefore, a thickness within the range of several nanometers to 1 µm is preferable.
The organic EL device which can be produced as described above emits light when a direct voltage of 5 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be employed.

### Example

### (1-1) Synthesis of Intermediate Product A1

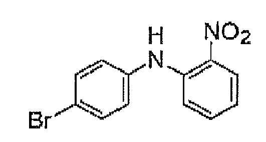

### Intermediate Product A1

39 g (0.27 mole) of 2-fluoronitrobenzene, 90 g (1.1 mole)of sodium acetate and 47 g (0.27 mole) of 4-bromoaniline were placed, followed by stirring at 120 degC for 8 hours, and then the resultant was dissolved in 300 ml of dichrolomethane, followed by washing with water and saturated salt water sequentially. The resultant was dried with the use of sodium sulfate anhydride, and then the solvent was removed by vacuum distillation. The resultant was refined by using a silica-gel column chromatography (developing solvent: dichloromethane), and then the crystal obtained was washed with methanol. 41 g of Intermediate Product A1 was obtained. Yield: 50%.

### (1-3) Synthesis of Intermediate Product A3

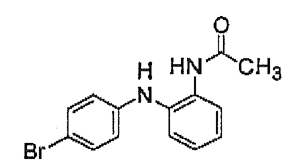

### Intermediate Product A3

13.5 g (51 mmol) of Intermediate Product A3 was dissolved in 100 ml of N-methylpyrrolidone and then 7.8 g (76 mmol) of acetic anhydride was dropped therein, followed by stirring at room temperature for 3 hours. After the reaction was completed, the reaction mixture was added into 500 ml of water. The precipitated solid was separated by filtration, and the solid was dried under vacuum pressure. 9.6 g of Intermediate Product A3 was obtained. Yield: 61 %.

### (1-4) Synthesis of Intermediate Product A4

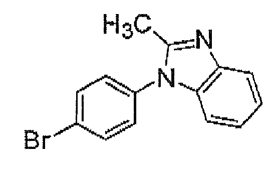

### Intermediate Product A4

9.6 g (31 mmol) of Intermediate Product A3 was dissolved in 100 ml of xylene, and 0.6 g (3.2 mmol) of p-toluenesulfonic acid monohydrate was added therein, followed by azeotropic dehydration for 8 hours under heating and refluxing. The reaction solution was cooled to room temperature, and then the resultant was refined by using a silica-gel column chromatography (developing solvent: dichloromethane), and then the crystal obtained was. 5.8 g of Intermediate Product A4 was obtained. Yield: 64 %.

### (1-5) Synthesis of Intermediate Product A5

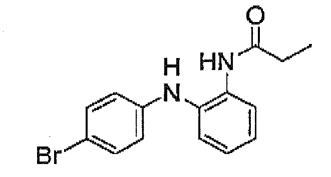

### Intermediate Product A5

4.5 g (17 mmol) of Intermediate Product A2 was dissolved in 50 ml of ethyl acetate, and then 2.6 g (26 mmol) of triethylamine and 2.4 g (25 mmol) of propionyl chloride were dripped therein, followed by stirring at room temperature for 3 hours. After the reaction was completed, the reaction mixture was added into 200 ml of water, followed by extraction with chloromethane. The organic layer obtained was washed with 5% hydrochloric acid solution and saturated salt water sequentially and the obtained was dried with sodium sulfate. The solvent was removed by using a rotary evaporator, and 5.5 g of Intermediate Product A5 was obtained. Yield: 100%.

### (1-6) Synthesis of Intermediate Product A6

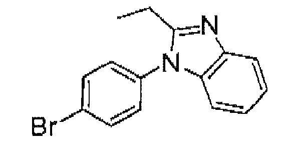

### Intermediate Product A6

Intermediate Product A6 of white crystal was obtained in a similar manner as Synthesis of Intermediate Product A4 except that Intermediate Product A5 was used in place of Intermediate Product A3. Yield: 1.6 g (31 %).

### (1-7) Synthesis of Intermediate Product A7

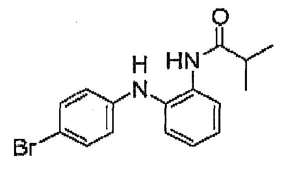

### Intermediate Product A7

Intermediate Product A7 of white crystal was obtained in a similar manner as Synthesis of Intermediate Product A5 except that isobutyl chloride was used in place of propionyl chloride. Yield: 5.0 g (90 %). (1-8) Synthesis of Intermediate Product A8

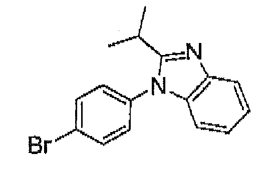

### Intermediate Product A8

Intermediate Product A8 of white crystal was obtained in a similar manner as Synthesis of Intermediate Product A4 except that Intermediate Product A7 was used in place of Intermediate Product3. Yield: 2.0 g (42 %).

### (1-9) Synthesis of Compound (1)

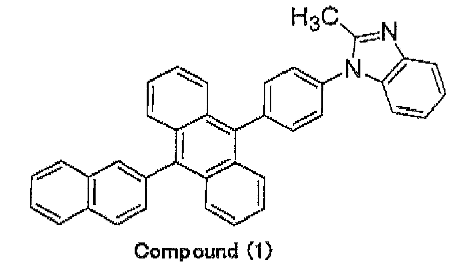

Under the argon gas current, into a three neck flask of 300 ml, 5.8 g (20 mmol) of Intermediate Product A4, 8.1 g (23 mmol) of 10-naphthalene-2-yl-anthracene-9-boronic acid, 0.47 g (0.4 mmol) tetrakistriphenylphosphine palladium (0), 70 ml of 1,2-dimethoxyethane and 35 ml (70 mmol) of 2M-sodium carbonate aqueous solution were placed, followed by heating and refluxing for 8 hours. After the reaction was completed, the organic layer was dried with use of magnesium sulfate, and then the solvent was removed by using a rotary evaporator. The crude crystal obtained was washed with 50 ml of toluene and 100 ml of methanol, and 6.9 g of pale-yellow powder was obtained, The powder was confirmed as Compound (1) by FD-MS (Field Desorption Mass Spectrometry) measurement.

### (1-10) Synthesis of Compound (2)

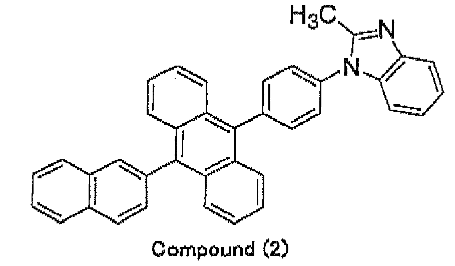

Compound (2) of pale-yellow powder was obtained in a similar manner as Synthesis of Compound (1) except that 10-naphthalene-1-yl-anthracene-9-botonic acid was used in place of 10-naphthalene-2-yl-anthracene-9-boronic acid. Yield: 3.1 g (58 %). The powder was conformed as Compound (2) by FD-MS (Field Desorption Mass Spectrometry) measurement.

### (1-11) Synthesis of Compound (3)

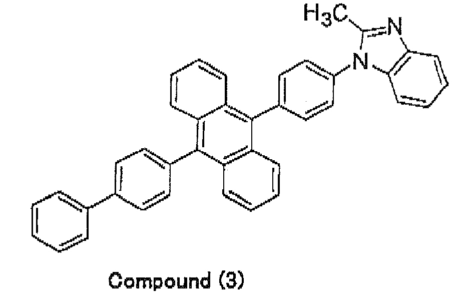

Compound (3) of pale-yellow powder was obtained in a similar manner as Synthesis of Compound (1) except that 4-biphenyl-anthracene-9-boronic acid was used in place of 10-naphthalene-2-yl-anthracene-9-boronic acid. Yield: 2.8 g (50 %). The powder was confirmed as Compound (3) by FD-MS (Field Desorption Mass Spectrometry) measurement.

### (1-12) Synthesis of Compound (4)

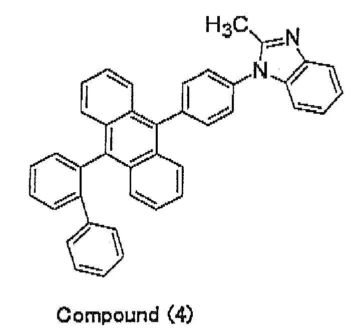

Compound (4) of pale-yellow powder was obtained in a similar manner as Synthesis of Compound (1) except that 2-biphenyl-anthracene-9-boronic acid was used in place of 10-naphthalene-2-yl-anthracene-9-boronic acid. Yield: 2.5 g (45 %). The powder was confirmed as Compound (4) by FD-MS (Field Desorption Mass Spectrometry) measurement.

### (1-13) Synthesis of Compound (5)

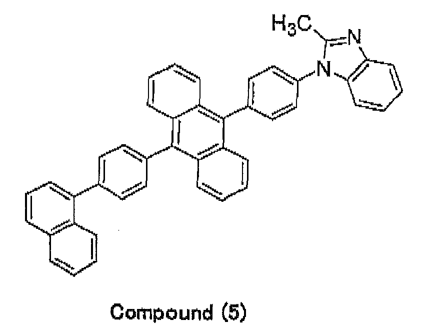

Compound (4) of pale-yellow powder was obtained in a similar manner as Synthesis of Compound (1) except that 4-(1-naphthyl)phenyl-anthracene-9-boronic acid was used in place of 10-naphthalene-2-yl-anthracene-9-boronic acid. Yield: 3.0 g (49 %). The powder was conformed as Compound (5) by FD-MS (Field Desorption Mass Spectrometry) measurement.

### (1-14) Synthesis of Compound (6)

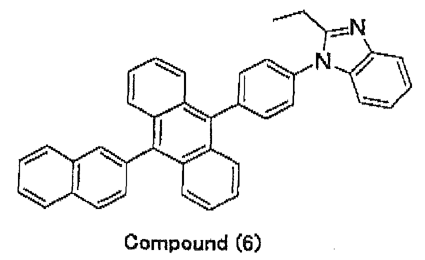

Compound (6) of pale-yellow powder was obtained in a similar manner as Synthesis of Compound (1) except that Intermediate Product A6 was used in place of Intermediate Product A4. Yield: 1.7 g (61 %). The powder was confirmed as Compound (6) by FD-MS (Field Desorption Mass Spectrometry) measurement.

### (1-15) Synthesis of Compound (7)

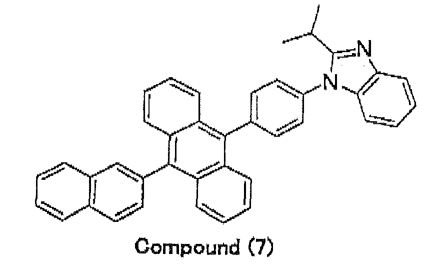

Compound (7) of pale-yellow powder was obtained in a similar manner as Synthesis of Compound (1) except that Intermediate Product A8 was used in place of Intermediate Product A4. Yield: 2.3 g (67 %). The powder was confirmed as Compound (7) by FD-MS (Field Desorption Mass Spectrometry) measurement.

### (2-1) Synthesis of Intermediate Product B1

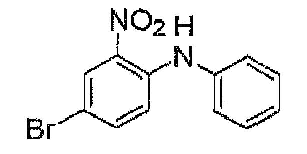

### Intermediate Product B1

50 g (0.18 mole) of 2,5-dibromonitrobenzene, 59 g (0.72 mole) of sodium acetate and 17 g (0.18 mole) of aniline were placed, followed by stirring at 120 degC for 8 hours, and then the resultant was dissolved in 300 ml of dichrolomethane, followed by washing with water and saturated salt water sequentially. The resultant was dried with the use of sodium sulfate anhydride, and then the solvent was removed by vacuum distillation. The resultant was refined by using a silica-gel column chromatography (developing solvent: dichloromethane), and then the crystal obtained was washed with methanol. 24 g of Intermediate Product B1 was obtained. Yileld: 45 %.

(2-2) Synthesis of Intermediate Product B2

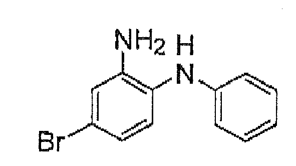

### Intermediate Product B2

20 g (68 ml) of Intermediate Product B1 was dissolved in 200 ml of tetrahydrofuran and while the resultant was stirred at a room temperature under argon atmosphere, 60 g (0.35 mol) of sodium hydrosulfite / 200 ml of water solution was dropped therein. In addition, 10 ml of methanol was added therein, followed by stirring for 3 hours. After the reaction solution became clear so that the reaction was completed, 200 ml of ethyl acetate was added and it was neutralized by adding sodium hydrocarbonate aqueous solution. Then, the organic layer was separated, followed by washing with saturated salt water, and then the resultant was dried with use of magnesium sulfate anhydride. The solvent was removed by vacuum distillation so as to obtain 18 g of Intermediate Product B2. Yield: 100 %.

### (2-3) Synthesis of Intermediate Product B3

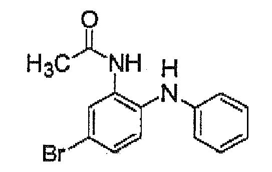

### Intermediate Product B3

18 g (68 mmol) of Intermediate Product B2 was dissolved in 200 ml of N-methylpyrrolidone, and then 10 g (98 mmol) of acetic acid anhydride was dropped therein, followed by stirring for 3 hours at room temperature. After the reaction was completed, the reaction product was added into 500 ml of water, and the precipitated solid was separated by filtration, followed by vacuum drying so as to obtain 9.7 g of Intermediate Product B3. Yield: 47 %.

(2-4) Synthesis of Intermediate Product B4

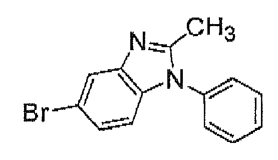

### Intermediate Product B4

9.7 g (32 mmol) of Intermediate Product B3 was dissolved in 100 ml of xylene, and 0.6 g (8.2 mmol) of p-toluenesulfonic acid hydrate was added therein, followed by azeotropic dehydration for 8 hours under heating and refluxing under nitrogen atmosphere. The reaction solution was cooled to room temperature, and then the resultant was refined by using a silica-gel column chromatography (developing solvent: dichloromethane), and then the crystal obtained was. 6.7 g of Intermediate Product B4 was obtained. Yield: 74%.

### (2-5) Synthesis of Intermediate Product B5

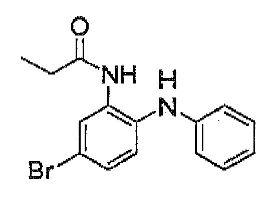

### Intermediate Product B5

7.0 g (27 mmol) of Intermediate Product B2 was dissolved in 80 ml of ethylacetate, followed by dropping 4.0 g (39 mmol) of triethylamine and 3.7 g (40 mmol) of propionyl chloride, and then the resultant was stirred at room temperature for 3 hours. After the reaction was completed, 300 ml of water was added into the reaction mixture, followed by extraction with dichloromethane. The organic layer obtained was washed with 5% hydrochloric acid and saturated salt water, and then dried with use of sodium sulfate. The solvent was removed by using a rotary evaporator so as to obtain 6.2 g of Intermediate Product B5. Yield: 74 %.

(2-6) Synthesis of Intermediate Product B6

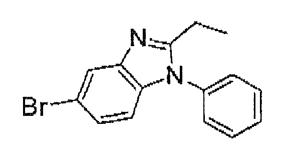

### Intermediate Product B6

Intermediate Product B6 of white crystal was obtained in a similar manner as Synthesis of Intermediate Product B4 except that Intermediate Product B5 was used in place of Intermediate Product B3. Yield: 2.8 g (47 %).

### (2-7) Synthesis of Intermediate Product B7

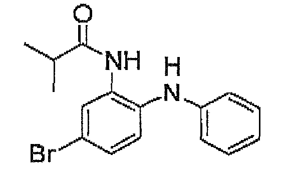

### Intermediate Product B7

Intermediate Product B7 of white crystal was obtained in a similar manner as Synthesis of Intermediate Product B5 except that isobutyl chloride was used in place of propionyl chloride. Yield: 8.2 g (92 %).

### (2-8) Synthesis of Intermediate Product B8

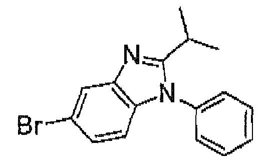

### Intermediate Product B8

Intermediate Product B8 of white crystal was obtained in a similar manner as Synthesis of Intermediate Product B4 except that Intermediate Product B7 was used in place of Intermediate Product B3. Yield: 1,1 g (14 %).

### (2-9) Synthesis of Compound (8)

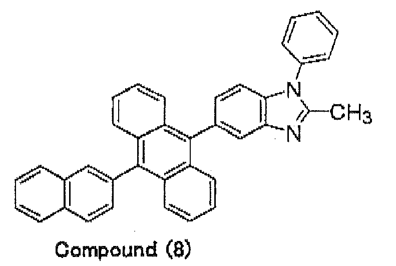

Under the argon gas current, into a three neck flask of 300 ml, 6.7 g (23 mmol) of Intermediate Product B4, 9.3 g (27 mmol) of 10-naphthalene-2-yl-anthracene-9-boronic acid, 0.54 g (0.5 mmol) of tetrakistriphenylphosphine palladium (0), 80 ml of 1,2-dimethoxyethane and 40 ml (80 mmol) of 2M-sodium carbonate aqueous solution were placed, followed by heating and refluxing for 8 hours. After the reaction was completed, the organic layer was washed with water and then dried with use of magnesium sulfate, followed by removing the solvent by using a rotary evaporator. The crude crystal obtained was washed with 50 ml of toluene and 100 ml of methanol, and 8.2 g of pale-yellow powder was obtained. The powder was confirmed as Compound (8) by FD-MS (Field Desorption Mass Spectrometry) measurement. Yield: 69 %.

### (2-10) Synthesis of Compound (9)

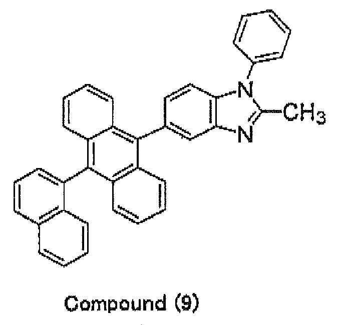

Compound (9) of pale-yellow powder was obtained in a similar manner as Synthesis of Compound (8) except that 10-naphthalene-1-yl-anthracene-9-boronic acid was used in place of 10-naphthalene-2-yl-anthracene-9-boronic acid. Yield: 2.5 g (47 %). The powder was confirmed as Compound (9) by FD-MS (Field Desorption Mass Spectrometry) measurement.

### (2-11) Synthesis of Compound (10)

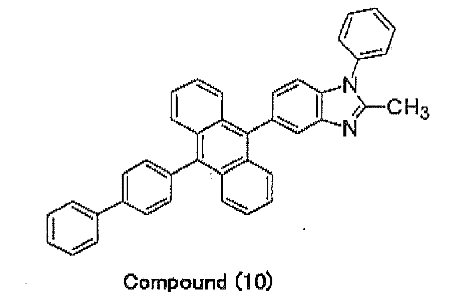

Compound (10) of pale-yellow powder was obtained in a similar manner as Synthesis of Compound (8) except that 4-biphenyl-anthracene-9-boronic acid was used in place of 10-naphthalene-2-yl-anthracene-9-boronic acid. Yield: 2.2 g (39 %). The powder was confirmed as Compound (10) by FD-MS (Field Desorption Mass Spectrometry) measurement.

### (2-12) Synthesis of Intermediate Product (11)

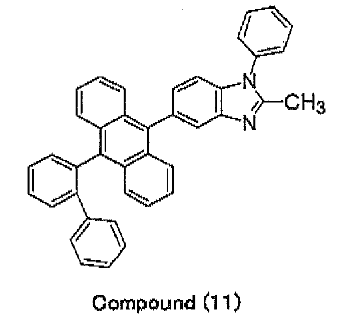

Compound (11) of pale-yellow powder was obtained in a similar manner as Synthesis of Compound (8) except that 2-biphenyl-anthracene-9-boronic acid was used in place of 10-naphthalene-2-yl-anthracene-9-boronic acid. Yield: 2.8 g (36 %). The powder was confirmed as Compound (11) by FD-MS (Field Desorption Mass Spectrometry) measurement.

### (2-13) Synthesis of Compound (12)

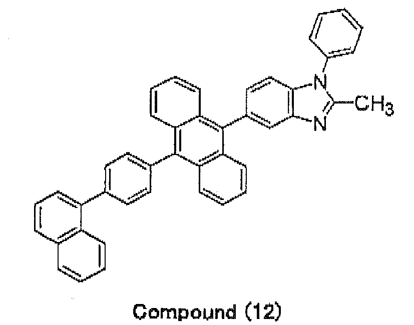

Compound (12) of pale-yellow powder was obtained in a similar manner as Synthesis of Compound (8) except that 4-(1-naphtyl)phenyl-anthracene-9-boronic acid was used in place of 10-naphthalene-2-yl-anthracene-9-boronic acid. Yield: 2.8 g (46 %). The powder was confirmed as Compound (12) by FD-MS (Field Desorption Mass Spectrometry) measurement.

### (2-14) Synthesis of Compound (13)

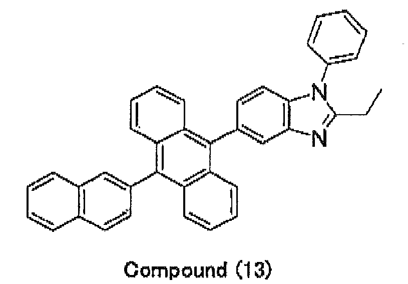

Compound (13) of pale-yellow powder was obtained in a similar manner as Synthesis of Compound (8) except that Intermediate Product B6 was used in place of Intermediate Product B4. Yield: 2.7 g (55 %). The powder was confirmed as Compound (13) by FD-MS (Field Desorption Mass Spectrometry) measurement.

(2-15) Synthesis of Compound (14) 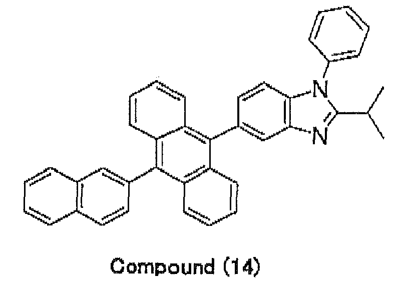 Compound (14) of pale-yellow powder was obtained in a similar manner as Synthesis of Compound (8) except that Intermediate Product B8 was used in place of Intermediate Product B4. Yield: 1.3 g (69 %). The powder was confirmed as Compound (14) by FD-MS (Field Desorption Mass Spectrometry) measurement.

### Example 1 (Fabrication of an organic EL device)

A glass substrate (manufactured by GEOMATEC Company) of 25 mm×75 mm×1.1 mm thickness having an ITO transparent electrode was cleaned by application of ultrasonic wave in isopropyl alcohol for 5 minutes and then by exposure to ozone for 30 minutes. The glass substrate having the transparent electrode lines which had been cleaned was attached to a substrate holder of a vacuum vapor deposition apparatus. On the surface of the cleaned substrate at the side having the transparent electrode, a film of N, N, N',N-tetra(-diamino-1,1'-biphenyl)diaminobiphenylene (referred to as "TPD232 film", hereinafter) having a thickness of 60 nm was formed so that the formed film covered the transparent electrode. The formed film of TPD232 worked as the hole injecting layer. Successively, a film of tetrakis-N-(4-biphenyl)benzidine with a film thickness of 20 nm (referred to as "TBB film", hereinafter) was formed over the film of TPD232. The TBB film worked as the hole transporting layer.
Further, a film having a thickness of 40 nm comprising the styryl derivative "DPVDPAN" represented by the following formula and the styrylamine derivative "S1" represented by the following formula with the film thickness ratio of DPVDPAN : S1= 40 : 2 was deposited on TBB film to form a light emitting layer emitting blue hue color.
On the film formed above, a film of Compound (1) having a thickness 20 nm was formed as an electron transporting layer. Subsequently, a film of LiF having 1 nm thickness was formed. On the LiF film, metallic aluminum of 150 nm was deposited to form a metal cathode and an organic EL device was fabricated.

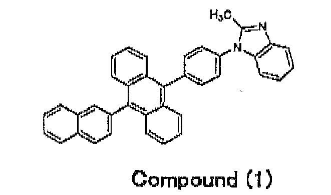

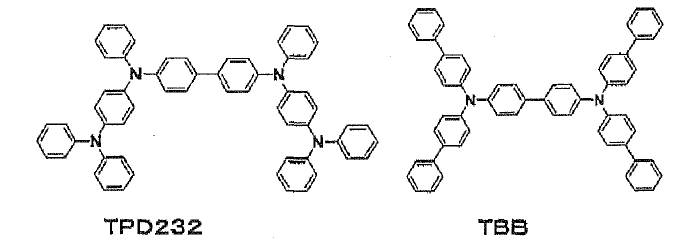

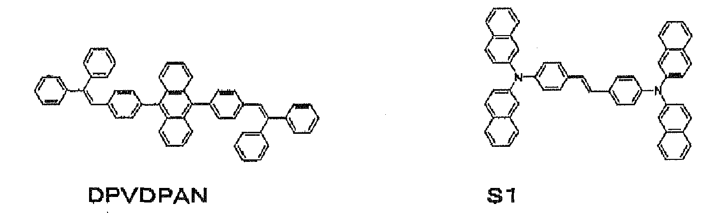

### Example 2

An organic EL device was fabricated similarly as Example 1 except that Compound (2) was used in place of Compound (1).

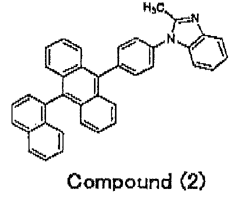

### Example 3

An organic EL device was fabricated similarly as Example 1 except that Compound (3) was used in place of Compound (1).

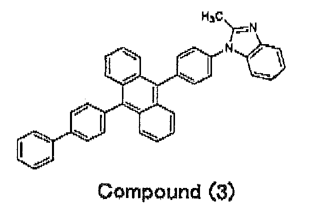

### Example 4

An organic EL device was fabricated similarly as Example 1 except that Compound (4) was used in place of Compound (1).

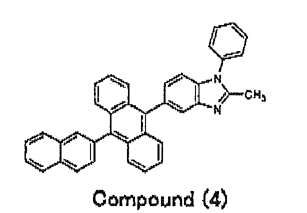

### Example 5

An organic EL device was fabricated similarly as Example 1 except that Compound (5) was used in place of Compound (1).

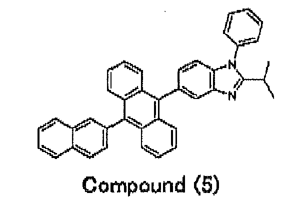

### Comparative Example 1

An organic EL device was fabricated similarly as Example 1 except that the following compound A described in International PCT publication No. WO 04/080975 A1 was used in place of Compound (1).

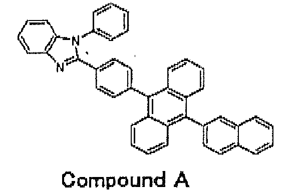

### Comparative Example 2

An organic EL device was fabricated similarly as Example 1 except that the following compound B described in International PCT publication No. WO 04/080975 A1 was used in place of Compound (1).

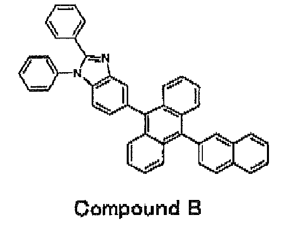

### Comparative Example 3

An organic EL device was fabricated similarly as Example 1 except that Alq (aluminum complex of 8-hydroxyquinoline) was used in place of Compound (1).

### Evaluation of Organic EL devices

The organic EL devices fabricated in Examples 1 to 5 and Comparative Examples 1 to 3 were measured on emission luminance, current efficiency and chromaticity under applying a direct current shown in Table 1, and each of the emitted color was observed. The results are shown in Table 1.

**Table 1**

| | Compound of Electron injecting layer | Electric Voltage (V) | Current Deisity (mA/cm2) | Emission Luminance (cd/m2) | Currant Effciency (cd/A) | Emitted Color |
|---|---|---|---|---|---|---|
| Example 1 | Compound (1) | 4.2 | 10.0 | 835,4 | 8,35 | blue |
| Example 2 | Compound (2) | 4.4 | 10.0 | 814.0 | 8.14 | blue |
| Example 3 | Compound (3) | 4.4 | 10.0 | 798.2 | 7.98 | blue |
| Example 4 | Compound (4) | 4.8 | 10.0 | 786.8 | 7.87 | blue |
| Example 5 | Compound (5) | 4.7 | 10.0 | 793.1 | 7.93 | blue |
| Comparative Example 1 | Compound A | 6.1 | 10.0 | 622.9 | 6.23 | blue |
| Comparative Example 2 | Compound B | 5.3 | 10.0 | 740.0 | 7.40 | blue |
| Comparative Example 3 | Alq | 6.2 | 10.0 | 480.3 | 4.80 | blue |

As shown from the results in Table 1, when the above compounds are used for a hole transporting material of an organic EL device, it is found to be able to produce a device exhibiting significantly high emission luminance and current efficiency.

## Claims

1. A heterocyclic derivative containing nitrogen represented by the following general formula (1): 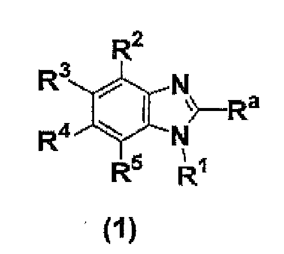 wherein, R¹ to R⁵ represents a hydrogen atom, a substituted or unsubstituted aryl group having 5 to 60 ring atoms, a substituted or unsubstituted heteroaryl group having 5 to 60 ring atoms, a substituted or unsubstituted pyridyl group, a substituted or substituted quinolyl group, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxyl group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group,
R^{a} represents a substituted or unsubstituted alkyl group having 1 to 50 carbon atoms, adjacent groups within R² to R⁵ may bond each other to form a aromatic ring and at least one of R¹ to R⁵ represents a substituent represented by the following general formula (2); 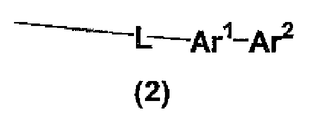 wherein, L represents a substituted or unsubstituted arylene group having 6 to 60 carbon atoms, a substituted or unsubstituted pyridinylene group, a substituted or unsubstituted quinolinylene group, or a substituted or unsubstituted fluorenylene group,
Ar¹ represents a substituted or unsubstituted arylene group having 6 to 60 carbon atoms, a substituted or unsubstituted pyridinylene group, or a substituted or unsubstituted quinolinylene group,
Ar² represents a hydrogen atom, a substituted or unsubstituted aryl group having 5 to 60 ring atoms, a substituted or unsubstituted pyridyl group, a substituted or unsubstituted quinolyl group, a substituted or unsubstituted alkyl group having 1 to 50 carbon atoms, a substituted or unsubstituted cycloalkyl group having 3 to 50 carbon atoms, a substituted or unsubstituted aralkyl group having 6 to 50 ring atoms, a substituted or unsubstituted alkoxy group having 1 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, an amino group substituted with a substituted or unsubstituted aryl group having 5 to 50 ring atoms, a halogen atom, a cyano group, a nitro group, a hydroxyl group or a carboxyl group.

2. The heterocyclic derivative containing nitrogen according to Claim 1, in which the general formula (1) is represented by the following general formula (1-a) or (1-b): 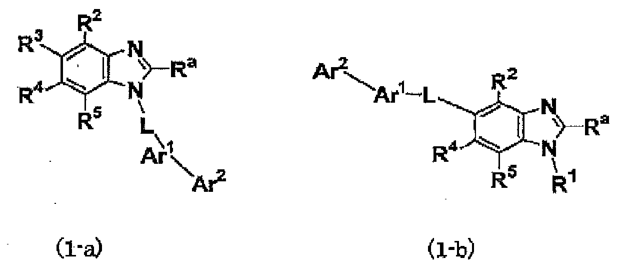 wherein, R¹ to R⁵, R^{a}, L, Ar¹ and Ar² each represents the same with all of the aforementioned general formula (1) respectively,
a pair of adjacent groups within R² to R⁵ may bond each other to form an aromatic ring.

3. The heterocyclic derivative containing nitrogen according to Claims 1 or 2, which is a material for an organic electroluminescence device.

4. The heterocyclic derivative containing nitrogen according to Claims 1 or 2, which is an electron injecting material or an electron transporting material for an organic electroluminescence device.

5. The heterocyclic derivative containing nitrogen according to Claims 1 or 2, which is a light emitting material for an organic electroluminescence device.

6. An organic electroluminescence device which comprises at least one organic thin film layer comprising a light emitting layer interposed between a pair of electrodes consisting of a cathode and an anode, wherein at least one of the organic thin film layers comprises the heterocyclic derivative according to Claims 1 or 2 singly or as its mixture component.

7. The organic electroluminescence device according to Claim 6, wherein, the organic thin film layer has an electron injecting layer or an electron transporting layer, and the electron injecting layer or the electron transporting layer contains the heterocyclic derivative containing nitrogen according to Claims 1 or 2 singly or as its mixture component.

8. The organic electroluminescence device according to Claim 6, which comprises one or more organic thin film layer comprising a light emitting layer interposed between a pair of electrodes consisting of an anode and a cathode, wherein the light emitting layer comprises the heterocyclic derivative containing nitrogen according to Claims 1 or 2 singly or as its mixture component,

9. The organic electroluminescence device according to Claim 7, **characterized by** that the device comprises the heterocyclic derivative containing nitrogen, which is an electron injecting material or an electron transporting material, according to Claims 1 or 2 comprising a reductive dopant

10. The organic electroluminescence device according to Claim 9, wherein a reductive dopant is one or more compound selected from the group comprising alkali metals, alkaline earth metals, rare earth metals, alkali metal oxides, alkali metal halides, alkaline earth metal oxides, alkaline earth metal halide, rare earth metal oxides, rare earth metal halides, organic complexes of alkali metals, organic complexes of alkaline earth metals and organic complexes of rare earth metals.
